# EUROPEAN PATENT SPECIFICATION

(11) **EP 4 403 647 B1**
(45) Date of publication and mention of the grant of the patent: **09.07.2025**
(21) Application number: 24175984.4
(22) Date of filing: 24.01.2023
(51) Int. Cl.: C12Q 1/6804, C12Q 1/6841

(54) **DETECTION OF PROXIMITY ASSAY PRODUCTS IN SITU**
IN-SITU-NACHWEIS VON PROXIMITÄTSASSAYPRODUKTEN
DÉTECTION DE PRODUITS DE DOSAGE DE PROXIMITÉ IN SITU

(30) Priority: 26.01.2022 US 202263303348 P
(43) Date of publication of application: 24.07.2024
(62) Divisional of application: 23702910.3
(73) Proprietor: MOLECULENT AB, 13334 Saltsjöbaden (SE)
(72) Inventor: ERICSSON, Olof John, Saltsjöbaden (SE); ROOS, Fredrik, Saltsjöbaden (SE)
(74) Representative: Williams Powell

(56) References cited:
- WO-A1-2018/160397
- BJÖRN KOOS ET AL: "Proximity-dependent initiation of hybridization chain reaction", NATURE COMMUNICATIONS, vol. 6, no. 1, 12 June 2015 (2015-06-12), XP055463141, DOI: 10.1038/ncomms8294

## Description

### BACKGROUND

Proximity ligation assay (PLA) permits detection of protein-protein interactions *in situ* (at distances of, e.g., < 40 nm) at endogenous protein levels. This method uses specific antibodies that identify (either directly or indirectly) two proteins of interest and takes advantage of specific DNA primers covalently linked to the antibodies. A hybridization step followed by rolling circle permit visualization of spots of proximity by fluorescence microscopy. Proximity ligation assay has been increasingly used to detect the interaction between two proteins with high sensitivity and specificity.

Alternative methods for performing proximity assays are highly desirable.

Koos et al. (2015) Nature Communications 6, 7294 disclose proximity-dependent initiation of hybridization chain reaction.

WO 2018/160397 discloses a circular proximity ligation assay.

### SUMMARY

According to an aspect of the present invention, there is provided an *in situ* proximity assay as specified in claim 1.

The targets that are bound by the binding agent-oligonucleotide conjugates may be proteinaceous. As such, in some embodiments, a binding agent-oligonucleotide conjugate may be composed of a binding agent (e.g., an antibody) that is conjugated to an oligonucleotide. Depending on the binding agent-oligonucleotide conjugates used, the present method can be employed to examine protein expression, post-translational modification, and protein-protein interactions, among other things.

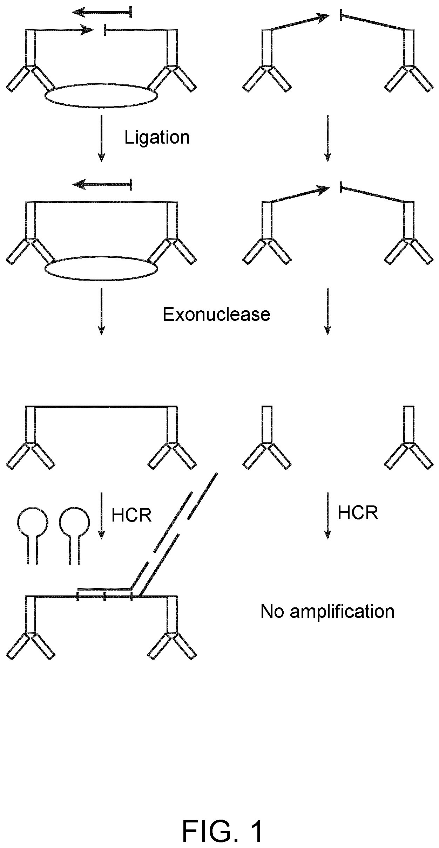

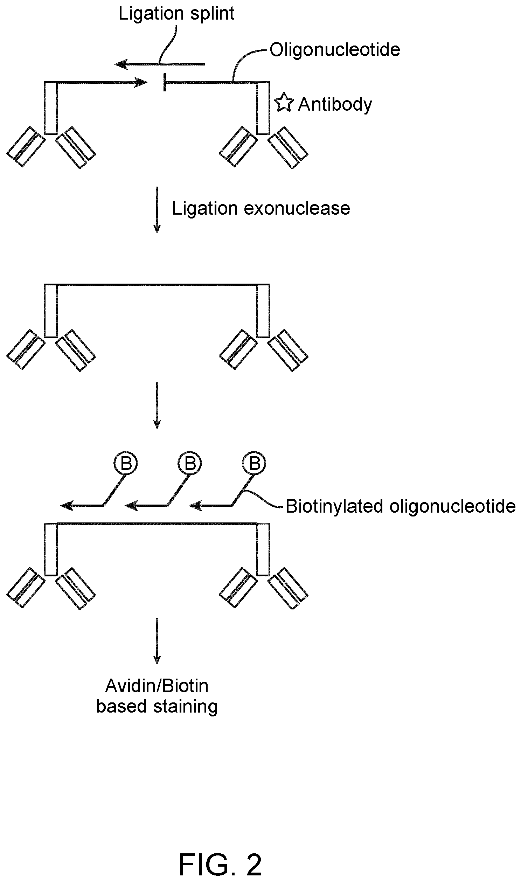

Examples of ways that the method may be implemented are shown in Figs. 1 and 2. Other implementations are described below.

### BRIEF DESCRIPTION OF THE FIGURES

The skilled artisan will understand that the drawings described below are for illustration purposes only. The drawings are not intended to limit the scope of the present teachings in any way.
**Fig. 1** illustrates one example of a way in which the present method may be implemented.
**Fig. 2** illustrates another example of a way in which the present method may be implemented.
**Fig. 3** illustrates a way a proximity assay can be implemented to make a reporter probe.
**Fig. 4** illustrates an exemplary staining method.
**Fig 5****.** Panel A: Detection of cytoplasmic keratin 5 and keratin 14 heterofilaments in squamous epithelial cells in tonsil by PLA. Hematoxylin stain is also added to visualize nuclei. Panel B: Only showing the PLA staining of keratin 5 and keratin 14 heterofilaments (in black) without HTX staining after color deconvolution.
**Fig. 6** Panel A: Detection of PCNA in nuclei of proliferating cells in tonsil by PLA. Hematoxylin stain is also added to visualize all nuclei. Panel B: Only showing the PLA staining of PCNA (in black) without HTX staining after color deconvolution.

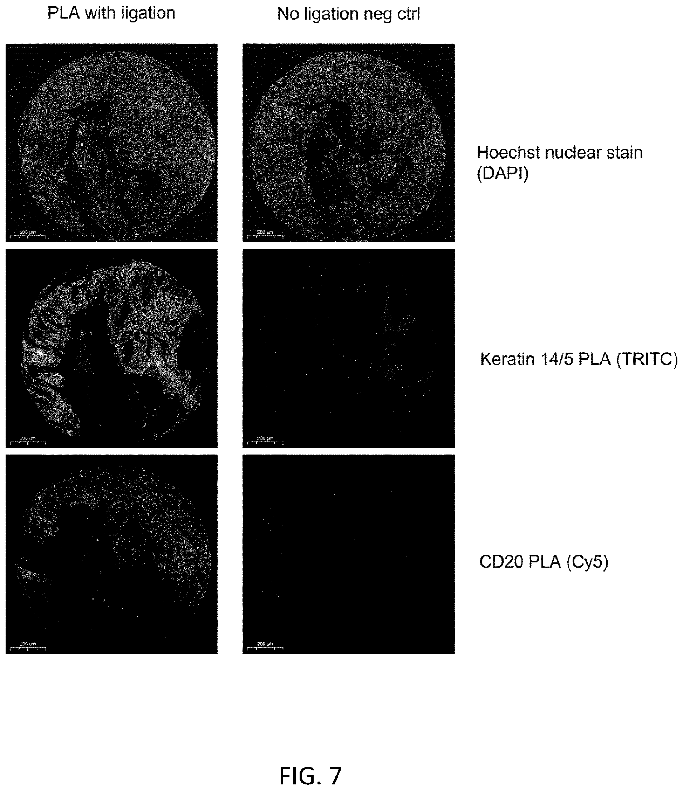

**Fig. 7****.** Duplex detection of keratin 5 and keratin 14 heterofilaments and CD20 by PLA in tonsil (right images). To the left is a technical negative control where the two ligation steps were ligation is omitted, illustrating that when ligation is omitted no PLA signal is detected. The top row shows images the tonsil tissue core in a TMA by staining all nuclei with Hoechst and imaging in DAPI. The middle row shows detection of keratin 5 and keratin 14 heterofilaments by PLA detected with HCR probes with ATTO565 fluorophores in TRITC channel. The lowest row shows detection of CD20 by PLA detected with HCR probes with ATTO647N fluorophores in Cy5 channel.
**Fig. 8****.** Duplex detection of keratin 5 and keratin 14 heterofilaments and CD20 by PLA in tonsil. The top row shows images the tonsil tissue core in a TMA by staining all nuclei with Hoechst and imaging in DAPI. The middle row shows detection of keratin 5 and keratin 14 heterofilaments by PLA detected with HCR probes with ATTO565 fluorophores in TRITC channel. The lowest row shows detection of CD20 by PLA detected with HCR probes with ATTO647N fluorophores in Cy5 channel.

### DEFINITIONS

Unless defined otherwise herein, all technical and scientific terms used in this specification have the same meaning as commonly understood by one of ordinary skill in the art to which this invention belongs. Although any methods and materials similar or equivalent to those described herein can be used in the practice or testing of the present invention, the preferred methods and materials are described.

Numeric ranges are inclusive of the numbers defining the range. Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention. Accordingly, the terms defined immediately below are more fully defined by reference to the specification as a whole.

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of ordinary skill in the art with the general meaning of many of the terms used herein. Still, certain terms are defined below for the sake of clarity and ease of reference.

As used herein, the term "multiplexing" refers to the simultaneous detection and/or measurement of multiple biological features of interest, e.g., protein epitopes, in a sample.

As used herein, the terms "antibody" and "immunoglobulin" are used interchangeably herein and are well understood by those in the field. Those terms refer to a protein consisting of one or more polypeptides that specifically binds an antigen. One form of antibody constitutes the basic structural unit of an antibody. This form is a tetramer and consists of two identical pairs of antibody chains, each pair having one light and one heavy chain. In each pair, the light and heavy chain variable regions are together responsible for binding to an antigen, and the constant regions are responsible for the antibody effector functions.

The terms "antibodies" and "immunoglobulin" include antibodies or immunoglobulins of any isotype and fragments of antibodies which retain specific binding to antigen, including, but not limited to, Fab, Fv, scFv, and Fd fragments, chimeric antibodies, humanized antibodies, minibodies, single-chain antibodies, and fusion proteins comprising an antigen-binding portion of an antibody and a non-antibody protein. Also encompassed by the term are Fab', Fv, F(ab')₂, and/ or other antibody fragments that retain specific binding to antigen, and monoclonal antibodies. Antibodies may exist in a variety of other forms including, for example, Fv, Fab, and (Fab')₂, as well as bi-functional (i.e. bi-specific) hybrid antibodies (e.g., Lanzavecchia et al., Eur. J. Immunol. 17, 105 (1987)) and in single chains (e. g., Huston et al., Proc. Natl. Acad. Sci. U.S.A., 85, 5879-5883 (1988) and Bird et al., Science, 242, 423-426 (1988)) (See, generally, Hood et al., "Immunology", Benjamin, N.Y., 2nd ed. (1984), and Hunkapiller and Hood, Nature, 323, 15-16 (1986)).

The term "specific binding" refers to the ability of a binding member to preferentially bind to another binding member that is present in a homogeneous mixture of different molecules.

In certain embodiments, the affinity between a binding members when they are specifically bound in a complex is characterized by a K_{D} (dissociation constant) of less than 10⁻⁶ M, less than 10⁻⁷ M, less than 10⁻⁸ M, less than 10⁻⁹ M, less than 10⁻⁹ M, less than 10⁻¹¹ M, or less than about 10⁻¹² M or less.

A "plurality" contains at least 2 members. In certain cases, a plurality may have at least 2, at least 5, at least 10, at least 100, at least 1000, at least 10,000, at least 100,000, at least 10⁶, at least 10⁷, at least 10⁸ or at least 10⁹ or more members. In certain cases, a plurality may have 2 to 100 or 5 to 100 members.

As used herein, the term "labeling" refers to a step that results in binding of a binding agent to specific sites in a sample (e.g., sites containing an epitope for the binding agent (e.g., an antibody) being used, for example) such that the presence and/or abundance of the sites can be determined by evaluating the presence and/or abundance of the binding agent. The term "labeling" refers to a method for producing a labeled sample in which any necessary steps are performed in any convenient order, as long as the required labeled sample is produced. For example, in some embodiments and as will be exemplified below, a sample can be labeled using labeled probes that can be detected to determine distribution of nucleic acids on a support.

As used herein, the term "planar biological sample" refers to a substantially flat, i.e., two-dimensional, material (e.g. glass, metal, ceramics, organic polymer surface or gel) that comprises cells or any combination of biomolecules derived from cells, such as proteins, nucleic acids, lipids, oligo/polysaccharides, biomolecule complexes, cellular organelles, cellular debris or excretions (exosomes, microvesicles). A planar biological sample can be made by, e.g., growing cells on a planar support, depositing cells on a planar support, e.g., by centrifugation, by cutting a three dimensional object that contains cells into sections and mounting the sections onto a planar support, i.e., producing a tissue section, adsorbing the cellular components onto a surface that is functionalized with affinity agents (e.g. antibodies, haptens, nucleic acid probes), introducing the biomolecules into a polymer gel or transferring them onto a polymer surface electrophoretically or by other means. The cells or biomolecules may be fixed using any number of reagents including formalin, methanol, paraformaldehyde, methanol: acetic acid, glutaraldehyde, bifunctional crosslinkers such as bis(succinimidyl)suberate, bis(succinimidyl)polyethyleneglycol, etc. This definition is intended to cover cellular samples (e.g., tissue sections, etc.), electrophoresis gels and blots thereof, Western blots, dot-blots, ELISAs, antibody microarrays, nucleic acid microarrays, etc. Depending on the specific technique used to prepare the section, a planar biological sample can have a thickness of anywhere from 20 to 50 nm and up to 5 to 10 µm.

As used herein, the term "tissue section" refers to a piece of tissue that has been obtained from a subject, optionally fixed, sectioned, and mounted on a planar support, e.g., a microscope slide.

As used herein, the term "formalin-fixed paraffin embedded (FFPE) tissue section" refers to a piece of tissue, e.g., a biopsy sample that has been obtained from a subject, fixed in formaldehyde (e.g., 3%-5% formaldehyde in phosphate buffered saline) or Bouin solution, embedded in wax, cut into thin sections, and then mounted on a microscope slide.

The phrase *"in situ"* as used here in refers to a specific position or location in a planar biological sample. For example, "a binding agent that is bound to the sample, *in situ,"* indicates that the binding agent is bound at a specific location in the planar biological sample.

A "diagnostic marker" is a specific biochemical in the body which has a particular molecular feature that makes it useful for detecting a disease, measuring the progress of disease or the effects of treatment, or for measuring a process of interest.

A "pathoindicative" cell is a cell which, when present in a tissue, indicates that the animal in which the tissue is located (or from which the tissue was obtained) is afflicted with a disease or disorder. By way of example, the presence of one or more breast cells in a lung tissue of an animal is an indication that the animal is afflicted with metastatic breast cancer.

The term "complementary site" is used to refer to an epitope for an antibody or aptamer, or nucleic acid that has a sequence that is complementary to an oligonucleotide probe. Specifically, if the binding agent is an antibody or aptamer, then the complementary site for the binding agent is the epitope in the sample to which the antibody or aptamer binds. An epitope may be a conformational epitope or it may be a linear epitope composed of, e.g., a sequence of amino acids. If the binding agent is an oligonucleotide probe, then the complementary site for the binding agent is a complementary nucleic acid (e.g., an RNA or region in a genome).

The term "epitope" as used herein is defined as a structure, e.g., a string of amino acids, on an antigen molecule that is bound by an antibody or aptamer. An antigen can have one or more epitopes. In many cases, an epitope is roughly five amino acids or sugars in size. One skilled in the art understands that generally the overall three-dimensional structure or the specific linear sequence of the molecule can be the main criterion of antigenic specificity.

A "subject" of diagnosis or treatment is a plant or animal, including a human. Non-human animals subject to diagnosis or treatment include, for example, livestock and pets.

As used herein, the term "incubating" refers to maintaining a sample and binding agent under conditions (which conditions include a period of time, one or more temperatures, an appropriate binding buffer and a wash) that are suitable for specific binding of the binding agent to molecules (e.g., epitopes or complementary nucleic acids) in the sample.

As used herein, the term "binding agent" refers to an agent that can specifically binds to complementary sites in a sample. Exemplary binding agents include oligonucleotide probes, antibodies, and aptamers. If antibodies or aptamers are used, in many cases they may bind to protein epitopes.

The terms "nucleic acid" and "polynucleotide" are used interchangeably herein to describe a polymer of any length, e.g., greater than about 2 bases, greater than about 10 bases, greater than about 100 bases, greater than about 500 bases, greater than 1000 bases, up to about 10,000 or more bases composed of nucleotides, e.g., deoxyribonucleotides, ribonucleotides or a combination thereof, and may be produced enzymatically or synthetically (e.g., PNA as described in U.S. Patent No. 5,948,902 and the references cited therein) and which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine, thymine, uracil (G, C, A, T and U respectively). DNA and RNA have a deoxyribose and ribose sugar backbone, respectively, whereas PNAs backbone is composed of repeating N-(2-aminoethyl)-glycine units linked by peptide bonds. In PNAs, various purine and pyrimidine bases are linked to the backbone by methylene carbonyl bonds. A locked nucleic acid (LNA), often referred to as an inaccessible RNA, is an RNA molecule comprising modified RNA nucleotides. The ribose moiety of an LNA nucleotide is modified with an extra bridge connecting the 2' oxygen and 4' carbon. The bridge "locks" the ribose in the 3'-endo (North) conformation, which is often found in A-form duplexes. LNA nucleotides can be mixed with DNA or RNA residues in the oligonucleotide whenever desired. The term "unstructured nucleic acid", or "UNA", is a nucleic acid containing non-natural nucleotides that bind to each other with reduced stability. For example, an unstructured nucleic acid may contain a G' residue and a C' residue, where these residues correspond to non-naturally occurring forms, i.e., analogs, of G and C that base pair with each other with reduced stability, but retain an ability to base pair with naturally occurring C and G residues, respectively. Unstructured nucleic acid is described in US20050233340.

As used herein, the term "oligonucleotide" refers to a multimer of at least 10, e.g., at least 15 or at least 30 nucleotides. In some embodiments, an oligonucleotide may be in the range of 15-200 nucleotides in length, or more. Any oligonucleotide used herein may be composed of G, A, T and C, or bases that are capable of base pairing reliably with a complementary nucleotide. 7-deaza-adenine, 7-deaza-guanine, adenine, guanine, cytosine, thymine, uracil, 2-deaza-2-thio-guanosine, 2-thio-7-deaza-guanosine, 2-thio-adenine, 2-thio-7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7- deaza purine, 5-methyl-cytosine, 5-propynyl-uridine, 5-propynyl-cytidine, 2-thio-thymine or 2-thio-uridine are examples of such bases, although many others are known. As noted above, an oligonucleotide may be an LNA, a PNA, a UNA, or a morpholino oligomer, for example. The oligonucleotides used herein may contain natural or non-natural nucleotides or linkages.

As used herein, the term "reading" in the context of reading a fluorescent signal, refers to obtaining an image by scanning or by microscopy, where the image shows the pattern of fluorescence as well as the intensity of fluorescence in a field of view.

As used herein, the term "signal generated by," in the context of, e.g., reading a fluorescent signal generated by addition of the fluorescent nucleotide, refers to a signal that is emitted directly from the fluorescent nucleotide or a signal that is emitted indirectly via energy transfer to another fluorescent nucleotide (i.e., by fluorescence resonance energy transfer (FRET)).

As used herein, the term "cleavable linker" refers to a linker containing a bond that can be selectively cleaved by a specific stimulus, e.g., a reducing agent such as TCEP or DTT.

The phrase "specific binding pair" as used herein comprises "a first binding member" and "a second binding member" that have binding specificity for one another. The binding members of a binding pair may be naturally derived or wholly or partially synthetically produced. A binding member has an area on its surface, or a cavity, which specifically binds to and is therefore complementary to a particular spatial and polar organization of the other binding member of a binding pair. Examples of specific binding pairs are antigen-antibody, biotin-avidin, hormone-hormone receptor, receptor-ligand, nucleic acids that hybridize with each other, and enzyme-substrate.

As used herein, the term "binding agent-oligonucleotide conjugate" or "binding agent conjugate" refers to a binding agent, e.g., an antibody, aptamer or oligonucleotide probe, that is non-covalently (e.g., via a streptavidin/biotin interaction) or covalently (e.g., via a "click" reaction (see, e.g., Evans Aus. J. Chem. 2007 60 : 384-395) or the like) linked to a single-stranded oligonucleotide in a way that the binding agent can still bind to its binding site. The nucleic acid and the binding agent may be linked via a number of different methods, including those that use a cysteine-reactive maleimide or halogen-containing group. The binding agent and the oligonucleotide may be linked proximal to or at the 5' end of the oligonucleotide, proximal to or at the 3' end of the oligonucleotide, or anywhere in-between. The linkage between a binding agent and the oligonucleotide in a binding agent-oligonucleotide conjugate can be cleavable so that the nucleic acid reaction product can be released from the corresponding binding agents via cleavage of the cleavable linker. As will be illustrated below, a binding agent-oligonucleotide conjugate can be composed of a single oligonucleotide, where one region of the polynucleotide (the "probe" part of the oligonucleotide which may be in the region of 15-50 bases in length hybridizes to a target nucleic acid in the sample (e.g., an RNA) and the other region does not hybridize to that target and is free to participate in the other reactions that are described herein.

The phrase "proximity assay" as used herein refers to assays in which a new DNA product (e.g., a ligation product or primer extension product) is produced only if two binding events are proximal. In a proximity assay, oligonucleotides are joined to target specific binding agents, such as antibodies, aptamers or oligonucleotide probes. The new DNA product can be produced by a variety of different ways. For example, the new DNA product can be produced by an initial enzymic reaction between one first oligonucleotide and another (by a reaction that, e.g., ligates one end of an oligonucleotide to a nearby oligonucleotide, extends one end of an oligonucleotide using a nearby oligonucleotide as a template, or joins one end of an oligonucleotide to a nearby oligonucleotide via a templated gap-fill/ligation reaction, etc.). Products are only produced when two binding agent-oligonucleotide conjugates are bound to sites that are proximal. Certain non-limiting examples of proximity assays include proximity extension assay (PEA) and proximity ligation assay (PLA). For clarity, a proximity assay may involve an initial enzymatic reaction (e.g., ligations, etc.) that occur between the oligonucleotides that are attached to the binding agents

The phrase "proximity assay reaction products" as used herein refers to the nucleic acids products of a proximity assay. As will be explained below, such products contain sequence from two oligonucleotides, or the sequence of one and the complement of another, where the sequences are joined together only in the presence of proximal binding events. The exact nature of a proximity assay reaction product may vary depending on how the assay is performed. In some embodiments, a proximity assay reaction product may be the product of an initial reaction that joins together two first oligonucleotides (by ligation or a gap-fill/ligation reaction). In these embodiments, the proximity assay reaction products contain the same sequences as the two oligonucleotides that have been joined together. In other embodiments, a proximity assay reaction product may be the product of an initial reaction that extends the 3' end of an oligonucleotide onto one another. In these embodiments, the proximity assay reaction products contain the same sequences as one of the oligonucleotides and the complement of the other.

The phrase "proximity extension assay" is intended to refer to a proximity assay that relies on primer extension, where one oligonucleotide uses the other as a template. In this assay, the oligonucleotides that are conjugated to two binding agent-oligonucleotide conjugates that are bound to sites that are proximal hybridize with each other via complementary sequences at the 3' end. The proximity extension assay then involves extending the 3' ends of the hybridized oligonucleotides, for example, using a polymerase, and using hybridized oligonucleotides as templates, to producing nucleic acid reaction products. The resulting nucleic acid reaction products (or their complements) indicate that the corresponding binding agent-oligonucleotide conjugates are bound to sites that are proximal. Certain details of PEA are described by Di Giusto et al. (2005), Nucleic Acids Research, 33(6, e64):1-7; Lundberg et al. (2011) and Nucleic Acids Research, Vol. 39, No. 15; and Greenwood et al. (2015), Biomolecular Detection and Quantification, Vol. 4:10-16.

The phrase "proximity ligation assay" or PLA is intended to refer to a proximity assay in which one oligonucleotide is ligated to another oligonucleotide. Such ligation can involve blunt end ligation of single stranded or double stranded oligonucleotides, splint mediated ligation of single stranded oligonucleotides, or ligation of double stranded oligonucleotides having complementary overhangs, for example, overhangs comprising restriction enzyme recognition sites. In certain splint mediated ligations, the oligonucleotides hybridize to a splint in a manner that leaves a gap between the two ends of the oligonucleotides. In such cases, proximity ligation assay involves sealing the gap using a polymerase in a "gap-fill" reaction and then ligating the 3' end of the extended oligonucleotide to the 5' end of the other oligonucleotide. Regardless of the method used to ligate the oligonucleotides, the nucleic acid reaction products resulting from the ligation are analyzed. The resulting nucleic acid reaction products indicate that the corresponding binding agent-oligonucleotide conjugates are bound to sites that are proximal. Certain details of PLA are described by Fredriksson et al. (2002), Nature Biotechnology, 20:473-477; Gullberg et al. (2004), PNAS, 101(22):8420-8424; Wang et al. (2021), Applied Microbiology and Biotechnology, Vol. 105, pages 923-935; Greenwood et al. (2015), Biomolecular Detection and Quantification, Vol. 4:10-16.

The term "proximal" or the phrase "proximally located target sites" as used herein with respect to the location of target sites mean that the target sites are sufficiently close so that the oligonucleotides attached to the binding agent-oligonucleotide conjugates that bind to the target sites hybridize to teach other. The target sites can be on the same molecule, for example, two epitopes of one protein. The target sites can also be on different molecules, for example, two epitopes of two different proteins. The target sites can be on different types of molecules, for example, any combination of protein, RNA, DNA, lipid, carbohydrate, etc. The distant between the sites that can be called "proximally located target sites" depends on length of oligonucleotides attached to the binding agent-oligonucleotide conjugates and the presence of any linkers between the binding agents and the oligonucleotides. Typically, proximally located target sites are located at a distance that is less 50 nm, for example, less than 30 nm, less than 20 nm, less than 10 nm, or less than 5 nm.

The phrase "planar support" as used herein refers to a support to which the nucleic acid reaction products from the analyzed planar biological sample are transferred. A wide variety of different substrates can be used as a planar support. The planar support can be made from any suitable support material, such as glass, modified and/or functionalized glass, hydrogels, films, membranes, plastics (including e.g., acrylics, polystyrene, copolymers of styrene and other materials, polypropylene, polyethylene, polybutylene, polyurethanes, Teflon.TM., cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

The phrase "three-dimensional support" as used herein is intended to refer to a three dimensional, permeable solid through which DNA molecules can travel. In many cases, a three-dimensional support can be a cross-linked matrix, e.g., a gel.

Other definitions of terms may appear throughout the specification.

### DETAILED DESCRIPTION

### General principles

As noted above, this disclosure provides, among other thigs, a method for performing an *in situ* proximity assay. In many embodiments, the method may comprise: (a) performing a proximity assay on one or more pairs of binding agent-oligonucleotide conjugates that are bound to the sample, *in situ,* to produce proximity assay reaction products; and (b) labeling the sites in the sample at which the proximity assay reaction products are produced, wherein the labeling comprises hybridizing an oligonucleotide to the proximity assay reaction products and does not involve primer extension.

An example of one implementation of this method is schematically illustrated in Figs. 1 and 2. In this example, oligonucleotides that are proximal to one another are ligated together *in situ* in a splinted ligation reaction to produce proximity assay reaction products. After a treatment with an exonuclease (to remove unligated oligonucleotides, any splint oligonucleotides and other nucleic acids that might be a source of background) the sample is hybridized with an oligonucleotide that hybridizes to the proximity assay reaction products, which is then detected. In some embodiments, the oligonucleotide my hybridize to the ligation junction such that oligonucleotide hybridizes to at least 8 or 10 nucleotides at the 5' end of one of oligonucleotides that are attached to and antibody, and to at least 8 or 10 nucleotides at the 3' end of another of oligonucleotides that are attached to and antibody. As shown, in some embodiments, the oligonucleotide may have a tail that does not hybridize to the proximity assay reaction products, and the tail can be detected by any convenient method, e.g., hybridization chain reaction (HCR). HCR, an isothermal enzyme-free nucleotide polymerization method using hairpin DNAs (see, e.g., Evanko Nature Methods 2004 1: 186). In some embodiments, the hairpin oligonucleotides may be conjugated to a fluorophore, thereby allowing the site of the reaction product to be determined (see, e.g., Tsuneoka Front. Mol. Neurosci., 12 May 2020). In these embodiments, the oligonucleotide seeds the amplification reaction. Alternative detection methods would be apparent.

Consistent with Figs. 1 and 2, in some embodiments, the method comprising treating the sample with one or more exonucleases between steps (a) and (b), thereby removing any nucleic acids that have free 3' and/or 5' ends, wherein the one or more exonucleases may comprise at least a 5' exonuclease and a 3' exonuclease. This step is not necessary in some embodiments.

In some embodiments, the proximity assay may be a proximity ligation assay. In these embodiments, in (b) the oligonucleotide may hybridize across the ligation junction.

In other embodiments, the assay may be a proximity extension assay. In these embodiments, some of the capture agents used in the assay may be linked to primers and some of the capture agents may be linked to template oligonucleotides that include one or more cleavable nucleotides (e.g., uracils). In these embodiments, the method may further comprise cleaving the template oligonucleotides at the cleavable nucleotides (e.g., at the uracils), between steps (a) and (b). This can be done using USER enzyme, for example.

The proximity assay reaction products can be detected by any suitable method that does not involve primer extension or ligation, or any reaction that requires a polymerase or ligase. In some embodiments, the oligonucleotide of (b) may be labeled, e.g., fluorescently labeled or labeled with an affinity group. If the oligonucleotide of (b) is fluorescently labeled then it can be directly detected optically, e.g., by microscopy. In many embodiments, however, a signal amplification system may be used for example, if the oligonucleotide of (b) is biotinylate, then method may comprise incubating the sample with a streptavidin/avidin-peroxidase conjugate (e.g., an HRP conjugate) and a detectable substrate that is cleaved by the conjugate. HRP catalyzes the conversion of chromogenic substrates (e.g., 3,3',5,5'-Tetramethylbenzidine; TMB), 3,3'-Diaminobenzidine; DAB, 2,2'-azino-bis(3-ethylbenzothiazoline-6-sulfonic acid; ABTS and others) into colored products, and produces light when acting on chemiluminescent substrates. For example, in some embodiments, the labeling may be done by an Avidin-Biotin Complex (ABC) method (see, e.g., Bratthauer et al, Methods Mol Biol. 2010;588:257-7) or a Labeled Streptavidin-Biotin (LSAB) method (see, e.g. Garzetti et al Anticancer Res. 1998 18:609-13). Any of these methods can involve depositing tyramide deposition. This embodiment is illustrated in Fig. 2.

Alternatively, the oligonucleotide may be unlabeled and may have a tail that does not hybridize to the proximity assay reaction products. In these embodiments, the method may comprise detecting the tail, e.g., by hybridization chain reaction. This embodiment is illustrated in Fig. 1. In this embodiment, the hairpin oligonucleotide may be fluorescently labeled.

As would be apparent, any embodiment may involve imaging the sample.

A proximity ligation assay may comprise a templated ligation of oligonucleotides of the binding agent-oligonucleotide conjugates using a splint. The ligation may or may not involve extending the 3' end of one of the oligonucleotides to bring it next to the 5' end of the other oligonucleotide. A proximity extension assay may comprise hybridizing complementary 3' ends of the oligonucleotides of the binding agent-oligonucleotide conjugates and extending the 3' ends of the oligonucleotides using the other hybridized oligonucleotides as templates.

As illustrated in Fig. 1, the method may comprise binding a tissue section with a plurality of binding agent-oligonucleotide conjugates and performing a proximity assay on the bound conjugates, *in situ.* As shown, the binding agent part of the conjugates may be an antibody. However, in other embodiments, the binding agent may be an aptamer or oligonucleotide probe. The proximity assay may be done using a variety of different methods, e.g., a proximity ligation assay (which results in a first product in which the ends of the oligonucleotides in conjugates that are bind to sites that are proximal become ligated together) or a proximity extension assay (which results in a first product in which one or both oligonucleotides is/are extended using the other as a template). As noted above, the proximity assay can be performed in many different ways. In some embodiments, the oligonucleotides of two binding agent-oligonucleotide conjugates that are bound to proximal sites may be ligated together to produce a first product. This ligation reaction may be splinted, but it does not have to be.

This approach could be used to perform spectral multiplex proximity ligation using hybridization chain reaction, or a controlled branched hybridization reaction where different seeding sequences are used to generate distinct sequences labeled with different fluorophores.

The assay can be designed to combine both single binder immunoassays with proximity ligation assays by adding antibodies with exonuclease resistant oligonucleotides that can be detected without ligation in a separate spectral channel using separate HCR sequences or different affinity based signal amplification systems.

Proteins detection and interaction detection could also be done in a low spectrally resolved multiplex by performing proximity ligation both for protein detection and interaction analysis. Splints can be designed to allow ligation of one PLA probe both to another PLA probe for the same protein and to a PLA probe targeting a potentially interacting protein. The ligation junctions can then be detected following ligation and exonuclease treatment using oligonucleotides complementary to the sequences formed by the ligation allowing these to prime a HCR or a affinity based immune staining.

In some embodiments, the proximity assay products may be detected by hybridization chain reaction, an affinity-based in situ amplification systems like biotin/avidin. (examples outlined in Janardhan et al, Toxicol Pathol. 2018 46: 488-510.) Multiplex immunohistochemistry approaches using e.g. oligonucleotides for labeling (see, e.g., Cancer Commun (Lond). 2020 Apr; 40(4): 135-153.).

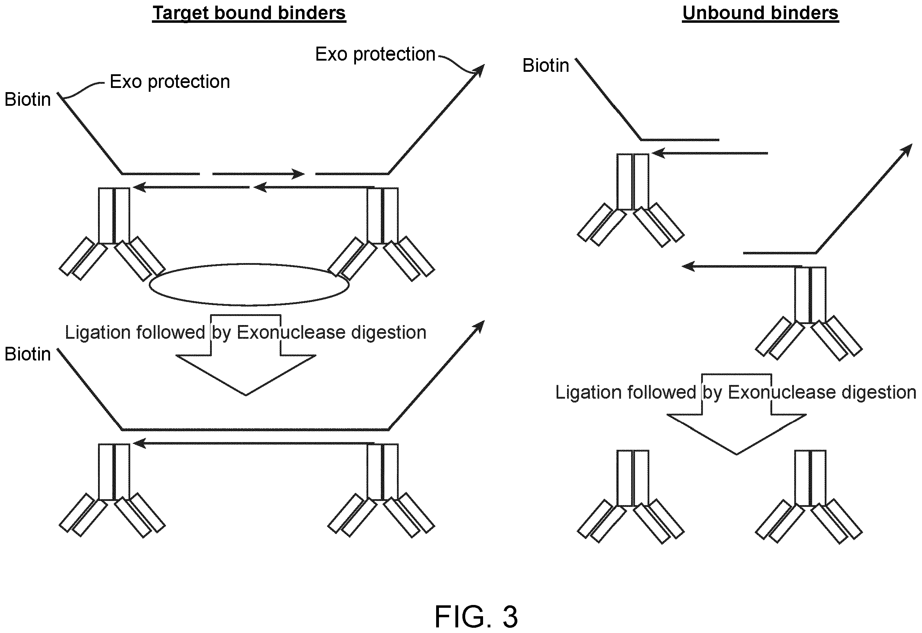

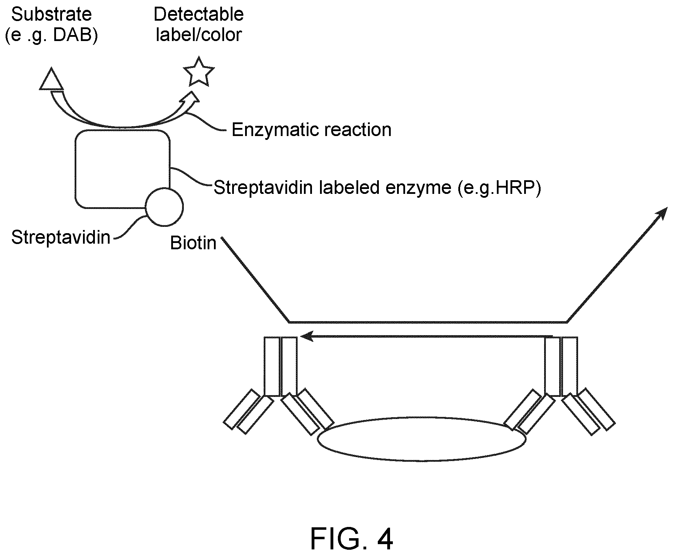

An embodiment of the method is illustrated in Fig. 3. In some embodiments and as illustrated, the method may comprise (a) labeling a biological sample with a plurality of conjugates that each comprise: i. a binding agent that binds to a site or sequence in the sample and ii. a first oligonucleotide; (b) ligating pairs of first oligonucleotides together, *in situ,* in the presence of a splint oligonucleotide to produce a first product, wherein the ligation is splinted by the splint oligonucleotide; (c) ligating the ends of the splint oligonucleotide to a first reporter oligonucleotide and a second reporter oligonucleotide, *in situ,* to produce a reporter probe, wherein: i. the ligation is splinted by the first product, ii. the first reporter oligonucleotide has a 5' end that is exonuclease resistant and a 3' hydroxyl; iii. the second reporter oligonucleotide has a 3' end that is exonuclease resistant and a 5' phosphate; and iv. the reporter probe is exonuclease resistant; and (d) treating the sample with an exonuclease to remove unligated reporter oligonucleotides, splint oligonucleotide, and first oligonucleotide. In some embodiments, the binding agent of (a) may be an antibody. As illustrated, the conjugates of (a) may comprise mixture of 3' conjugates in which the first oligonucleotide is joined to a binding agent by the 3' end and 5' conjugates in which the first oligonucleotide is joined to a binding agent by the 5' end. In some embodiments, the first reporter oligonucleotide is 5' biotinylated. In these embodiments and as illustrated in Fig. 4, the method may further comprise (e) incubating the sample with a streptavidin/avidin-peroxidase conjugate and a detectable substrate that is cleaved by the peroxidase, to stain the sample; and (f) imaging the sample to detect the staining. The staining is done by an Avidin-Biotin Complex (ABC) method or a Labeled Streptavidin-Biotin (LSAB) method for example. Alternatively, the method may comprise: (e) transferring the reporter probe into or onto a support in a way that preserves the spatial relationship of the proximity assay reaction products in the biological sample; and (f) detecting the reporter probe on the support by hybridization of a labeled probe to the reporter probe. In these embodiments, at least one of the reporter oligonucleotides may have a tail that does not hybridize to the first product and, in step (f) the labeled probe may hybridize with the tail of a reporter oligonucleotide in the reporter probe. Transfer and labeling protocols may be adapted from WO2022269543.

In any embodiment, the biological sample may be a tissue section.

### Binding Agents

A binding agent can be an antibody or an antigen binding fragment of an antibody, such as Fab, Fv, scFv, F(ab')₂, and Fd. A binding agent can also be a scaffold protein evolved for affinity like and Affibody or similar affinity proteins.

In some cases, an antibody against an antigen is a monoclonal antibody.

In one embodiment, an antibody against an antigen is a split polyclonal antibody. A split polyclonal antibody is produced by raising polyclonal antiserum against an antigen and splitting the antiserum into two portions. Oligonucleotides having a specific sequence are conjugated to antibodies against the antigen in one portion of the polyclonal antiserum and oligonucleotides having a different specific sequence are conjugated to the antibodies against the antigen in the other portion.

The oligonucleotide can be double stranded, single stranded or a combination thereof.

A binding agent can also be an aptamer that specifically binds to a protein, carbohydrate, or even small molecule.

Moreover, a binding agent can be an oligonucleotide that specifically binds to a target sequence, such as a specific target sequence in an RNA or DNA. An oligonucleotide binding agent can specifically bind to a target RNA, such as a messenger RNA (mRNA), transfer RNA (tRNA), or ribosomal RNA (rRNA). An oligonucleotide binding agent can also specifically bind to a target DNA, such as chromosomal DNA or extra-chromosomal DNA. Extra-chromosomal DNA can be an organelle DNA, such as mitochondrial DNA or chloroplast DNA.

### Binding Target sites

The binding agents can specifically bind to binding target sites, such as site on a protein, RNA, DNA, carbohydrate, proteoglycans, lipids, and other biomolecules.

The sites to which the binding agents bind may be on the same protein or on different proteins. For example, the binding agents may bind to different epitopes in the same protein. In some cases, one of the binding agents used in the proximity assay may binds to a site in a protein that is not post-translationally modified, whereas the other binding agent may specifically binds to the same protein at a site that is post-translationally modified. The post-translational modification can be for example phosphorylation, glycosylation, ubiquitination, nitrosylation, methylation, acetylation, and lipidation, although many others types of post-translational are known.

As noted above, an RNA binding target can be any type of RNA including mRNA, tRNA, non-coding RNA, or rRNA.

Also, a DNA binding target can be chromosomal DNA or extra-chromosomal DNA. Extra-chromosomal DNA can be an organelle DNA, such as mitochondrial DNA or chloroplast DNA.

In some cases, the oligonucleotide probes can be used to detect mutations in the DNA. In such cases, the target DNA is converted into single stranded without disrupting the tissue and other molecules. For example, specific targets in the DNA molecules can be converted into single stranded state by using nicking enzymes or CRISPR-based targeting. The single stranded DNA so produced can be digested, for example, using 3' or 5' specific exonuclease, to leave only one strand of the target DNA for the analysis of the mutation.

Target molecules can also be of viral or bacterial origin.

### Proximity Ligation Assay

In some embodiments, step (a) comprises PLA. Any suitable method can be used to ligate the oligonucleotides conjugated to the binding agent-oligonucleotide conjugates in a PLA. For example, the oligonucleotides from binding agent-oligonucleotide conjugates can be ligated together via: a non-templated ligation of single stranded ends of the nucleic acids, a non-templated ligation of double stranded ends of the nucleic acids, a templated ligation using a splint, or overhang mediated ligation of double stranded nucleic acids using complementary overhangs.

In one embodiment, PLA comprises contacting the biological sample with a first target specific binding agent-oligonucleotide conjugate comprising a first target oligonucleotide and a second target specific binding agent-oligonucleotide conjugate comprising a second target oligonucleotide. Plurality of pairs of first and second target specific binding agent-oligonucleotide conjugates can also be used in a multiplex reaction.

In a pair of target specific binding agent-oligonucleotide conjugates, the first target oligonucleotide has a free 3' end and comprises from the 5' end: one or more barcodes unique for the first target and a first splint hybridization region; and the second target oligonucleotide has a free 5' end and comprises from the 3' end: one or more barcodes unique for the second target and a second splint hybridization region.

Upon binding of the pairs of target specific binding agent-oligonucleotide conjugates to the corresponding target sites, the biological sample is contacted with a splint oligonucleotide that hybridizes at two ends with the first and the second oligonucleotides that are brought proximal to each other via the binding of the first target specific binding agent to the first target site and the second target specific binding agent to the second target site.

The splint oligonucleotide can bring together the 5' and 3' ends of the first and second oligonucleotides, in which case, the two oligonucleotides can be ligated to produce a ligated oligonucleotides. The splint oligonucleotide can be designed so that the 3' end of one oligonucleotide is not proximal to the 5' end of the other oligonucleotide. In such cases, the 3' end can be extended, for example, using a polymerase to extend the 3' end of the first oligonucleotide towards the 5' end of the second oligonucleotide. The two oligonucleotides can then be ligated to produce the ligated oligonucleotide.

Thus, in some cases, the ligation assay may comprise: (i) labeling the planar biological sample with multiple pairs of binding agent-oligonucleotide conjugates, (ii) hybridizing splint oligonucleotides to the sample after (i), wherein the splint oligonucleotides hybridize with the ends of the oligonucleotides in different conjugates, and (iii) ligating together the ends of any oligonucleotides in the conjugates that are hybridized to the same splint oligonucleotide, to produce the nucleic acid reaction products.

In some embodiments, the ligation assay may comprise ligating the oligonucleotides of the binding agent-oligonucleotide conjugates via: a templated or non-templated ligation of single stranded ends of the oligonucleotides, a non-templated ligation of double stranded ends of the oligonucleotides, or overhang mediated ligation of double stranded oligonucleotides using complementary overhangs. Templated ligation embodiments may be accomplished using a ligation splint, wherein the splint is designed so that the 3' end of a first oligonucleotide of a first binding agent-oligonucleotide conjugate is next to the 5' end of a second oligonucleotide of a second binding agent-oligonucleotide conjugate, and wherein the method comprises ligating the 5' and 3' ends of the first and second oligonucleotides.

Oligonucleotides can also be joined to one another via a gap-fill/ligation reaction, in which two oligonucleotides hybridize to the opposing ends of a template, and the gap is filled by polymerization and the nick is sealed by ligation.

### Proximity Extension Assay (PEA)

In some embodiments, step (a) comprises PEA. Any suitable method can be used to produce nucleic acid reaction products from the oligonucleotides from the binding agents-oligonucleotide conjugates. In one embodiment, PEA comprises contacting the biological sample with a first target specific binding agent-oligonucleotide conjugate comprising a first oligonucleotide and a second target specific binding agent-oligonucleotide conjugate comprising a second oligonucleotide. Plurality of pairs of first and second target specific binding agent-oligonucleotide conjugates can also be used in a multiplex reaction.

In a pair of specific binding agent-oligonucleotide conjugates, the free 3' ends of the first and the second oligonucleotides have sequences that are complementary to each other and, hence, the free ends hybridize to each other. These free 3' ends can be extended, for example, using a polymerase, to create double stranded oligonucleotides that contain sequences of both the first and the second oligonucleotides.

Thus, in some cases, PEA comprises:
(i) labeling the planar biological sample with multiple pairs of binding agent-oligonucleotide conjugates,
(ii) hybridizing complementary 3' ends of the oligonucleotides and extending the 3' ends of the oligonucleotides using the other hybridized oligonucleotides as templates to produce the nucleic acid reaction products.

### Hybrid assays

In some embodiments, multiple combinations of interactions are analyzed using the method disclosed herein and a combination of PLA and PEA is used to produce the nucleic acid reaction products.

For example, nucleic acid reaction products are produced from certain interactions using PLA and nucleic acid reaction products are produced from certain other interactions using PEA. Certain details of PLA and PEA are described above and can be used in the hybrid methods envisioned herein.

### RNA detection using ligation assay

In some cases, the method involves detecting RNA using a ligation assay. Particularly, the reporter polynucleotides can be designed that hybridize with certain sequences in the target RNA. The probes can have tails containing barcodes. In some cases, the oligonucleotide probes comprise DNA nucleotides, except towards the ligation site, where the oligonucleotides comprise RNA nucleotides. Thus, the oligonucleotide probes can be a hybrid of DNA and RNA nucleotides. Alternatively, the reporter polynucleotides can comprise hairpin structures so that the oligonucleotides ligate with each other upon being brought together via the target RNA.

### Proximity assay with three or more binding agents

In some cases, proximity assay is performed with three or more binding agents. An example of such assay is described by Schallmeiner et al. (2007), Nat. Methods.;4(2):135-7*.*

In some cases, three binding agent-oligonucleotide conjugates are used, wherein a first binding agent-oligonucleotide conjugate is conjugated to a first oligonucleotide, the second binding agent-oligonucleotide conjugate is conjugated to a second oligonucleotide, and the third binding agent-oligonucleotide conjugate is conjugated to a splint oligonucleotide. If the three oligonucleotides are brought proximal to each other via binding of the three binding agents to proximal binding targets, the splint oligonucleotide hybridizes with the first and the second oligonucleotides, which can be ligated to produce nucleic acid reaction product. Thus, the production of the nucleic acid reaction product indicates that the oligonucleotides are conjugated to binding agents that are bound to sites that are proximal.

### Exonuclease digestion

In any embodiment, the method may comprise a digestion with one or more exonucleases (e.g., both exonuclease I and exonuclease III, although other one or more other exonucleases, e.g., exonuclease T, exonuclease V, exonuclease VII, T5 exonuclease or T7 exonuclease could be used instead in some cases) to remove unligated reporter oligonucleotides and other single stranded nucleic acids. This digestion can be done any time after the initial proximity assay reaction products have been produced. For example, the digestion may be done in situ. In these embodiments, the oligonucleotides that are used in the proximity assay (e.g., the first oligonucleotides that are attached the binding agents, or the reporter oligonucleotides) may be designed to be produce exonuclease resistant products, which allows those products to survive the exonuclease step. Following ligation, the ligation product will be joined to the antibodies and not comprise free 3 or 5' ends rendering it resistant to exonuclease digestion by enzymes degrading from 3 and or 5' ends. Oligonucleotides can be made exonuclease-resistant by addition of an exonuclease-resistant linkage, such as a phosphorothioate linkage, although other linkages can be used. In alternative embodiments, two antibodies with free 3' arms mediate templated ligation of an exonuclease resistant oligonucleotide on each other. In another embodiment PEA is performed with exonuclease resistant nucleotides.

### Signal amplification using redundant ligation events

In order to generate more signal from rare binding events, several nucleic acid reaction products can be generated from each binding event. For protein targets this can be achieve using binding agents that are each conjugated to several oligonucleotides. This, in turn, generates several assay products in the proximity assay. For RNA and DNA targets multiple probe sets can be designed targeting each RNA molecule or DNA loci so that each target produces many nucleic acid reaction products. At least 2 or more or at least 5 or more or at least 10 or more nucleic acids can be used per binder directly or indirectly associated with the binding agent. At least 2 or more or at least 5 or more or at least 10 or more at least 20 or more probe pairs can be used to target RNA or DNA sequences.

### Kits

Also provided by this disclosure are kits that contain reagents for practicing the subject method, as described above. These various components of a kit may be in separate vessels or mixed in the same vessel.

The various components of the kit may be present in separate containers or certain compatible components may be pre-combined into a single container, as desired.

In addition to the above-mentioned components, the subject kit may further include instructions for using the components of the kit to practice the subject method.

### Utility

The methods and compositions described herein find general use in a wide variety of applications for analysis of planar biological samples (e.g., in the analysis of tissue sections, sheets of cells, or spun-down cells). The method may be used to analyze any tissue, including tissue that has been clarified, e.g., through lipid elimination, for example. The sample may be prepared using expansion microscopy methods (see, e.g., Chozinski et al. Nature Methods 2016 13: 485-488), which involves creating polymer replicas of a biological system created through selective co-polymerization of organic polymer and cell components. The method can be used to analyze spreads of cells, exosomes, extracellular structures, biomolecules deposited on a solid support or in a gel (Elisa, western blot, dot blot), whole organism, individual organs, tissues, cells, extracellular components, organelles, cellular components, chromatin and epigenetic markers, biomolecules and biomolecular complexes, for example. The binding agents may bind to any type of molecule, including proteins, lipids, polysaccharides, proteoglycans, metabolites, nucleic acid, or artificial small molecules or the like. The method may have many biomedical applications in screening and drug discovery and the like. Further, the method has a variety of clinical applications, including, but not limited to, diagnostics, prognostics, disease stratification, personalized medicine, clinical trials and drug accompanying tests.

The field of spatial analysis technology, the disclosure aims to provide highly multiplex readout of protein-protein interactions and protein modifications *in situ.* The disclosure also allows single molecule analysis of proteins, protein post-translational modifications, and protein interactions.

The methods disclosed herein could also be used to analyze RNAs or RNA interactions between RNAs and other molecules, such as proteins, in a single assay format.

In some cases, the methods disclosed herein could be used to analyze target RNAs. For example, as discussed above, an RNA target from a planar biological sample can be directly copied into a reporter polynucleotide using reporter probes. Particularly, a proximity assay is not performed to produce a nucleic acid reaction product but the RNA target is used as a template to produce a reporter polynucleotide. Such step could for example be performed before contacting the sample with binding agents since antigen retrieval steps required for protein analysis may damage RNA but not DNA, or simultaneously with the introduction of detection oligonucleotides ligating to the first products generated by joining the nucleic acids of the binding agents.

Moreover, the methods disclosed herein could be used to analyze interactions of RNA with other biomolecules, such as RNA, protein, DNA, carbohydrates, lipids, etc. In certain such embodiments, proximity assay can be conducted using one binding agent targeting an RNA and another binding agent targeting a protein, carbohydrate, or lipid. Proximity assay can also be conducted using one binding agent targeting an RNA and another binding agent targeting a different RNA. Such embodiments can be used to analyze interaction of a target RNA to any other biomolecule for which there a specific binding agent is available.

In some cases, the method disclosed herein can be used to identify target sites that are located proximal to each other. For example, a first binding agent-oligonucleotide conjugate binds to a first site and a second binding agent-oligonucleotide conjugate binds to a second site. When the first site and the second site are proximal, the oligonucleotides are brought close to each other. Therefore, the production of a nucleic acid from the oligonucleotides conjugated to the first and the second binding agent-oligonucleotide conjugates indicates that the oligonucleotides are conjugated to binding agents that are bound to sites that are proximal.

Thus, in certain cases, the method disclosed herein can be used to: determine where specific protein proteins are located in a planar biological sample. In these embodiments, the binding agents bind to different sites on the same protein.

In some cases, the method disclosed herein can also be used to identify where protein-protein interactions occur. In these embodiments, the binding agents bind to different proteins.

Since the relative proximity of targets depends on the absolute concentration and the amount of signal generated from each interaction depends on additional efficiency factors like binding affinity and chemical and enzymatic efficiencies relating the relative signals within a multiplex experiment to each other will be advantageous. For example using reference proteins, RNA or DNA targets or relating the signal from the single individual proteins to the signal from the interaction of the proteins. The signals can for example be analyzed per cell, among a group of cells, for a cell-type determined by the presence of cellular markers or by area.

Also, in some cases, the method disclosed herein can be used to determine post-translation modification of a biomolecule, such as a protein. In certain such embodiments, one binding agent binds to the post translational modification or to an epitope covering both the post translational modification and the target protein and the other binding agent binds to a different site in the same protein. The production of a nucleic acid from the oligonucleotides conjugated to the first and the second binding agent-oligonucleotide conjugates indicates that the protein has sites that are post-translationally modified. By using binding agents specific for general post translational modifications, the presence of such modifications across a large number of proteins can be interrogated. Signal can advantageously be analyzed in a relative manner to normalize away the effect of global presence of modifications impact of protein concentrations and assay efficiencies.

In particular embodiments, the sample may be a section of a tissue biopsy obtained from a patient. Biopsies of interest include both tumor and non-neoplastic biopsies of skin (melanomas, carcinomas, etc.), soft tissue, bone, breast, colon, liver, kidney, adrenal, gastrointestinal, pancreatic, gall bladder, salivary gland, cervical, ovary, uterus, testis, prostate, lung, thymus, thyroid, parathyroid, pituitary (adenomas, etc.), brain, spinal cord, ocular, nerve, and skeletal muscle, etc.

In certain embodiments, binding agents specifically bind to biomarkers, including cancer biomarkers, that may be proteinaceous. Exemplary cancer biomarkers, include, but are not limited to carcinoembryonic antigen (for identification of adenocarcinomas), cytokeratins (for identification of carcinomas but may also be expressed in some sarcomas), CD15 and CD30 (for Hodgkin's disease), alpha fetoprotein (for yolk sac tumors and hepatocellular carcinoma), CD117 (for gastrointestinal stromal tumors), CD10 (for renal cell carcinoma and acute lymphoblastic leukemia), prostate specific antigen (for prostate cancer), estrogens and progesterone (for tumor identification), CD20 (for identification of B-cell lymphomas) and CD3 (for identification of T-cell lymphomas).

The above-described method can be used to analyze cells from a subject to determine, for example, whether the cell is normal or not or to determine whether the cells are responding to a treatment. In one embodiment, the method may be employed to determine the degree of dysplasia in cancer cells. In these embodiments, the cells may be a sample from a multicellular organism. A biological sample may be isolated from an individual, e.g., from a soft tissue. In particular cases, the method may be used to distinguish different types of cancer cells in FFPE samples.

The method described above finds particular utility in examining samples using a plurality of antibodies or antibody pairs, each antibody or antibody pair recognizing a different marker. Examples of cancers, and biomarkers that can be used to identify those cancers, are shown below. In these embodiments, one does not need to examine all of the markers listed below to make a diagnosis.

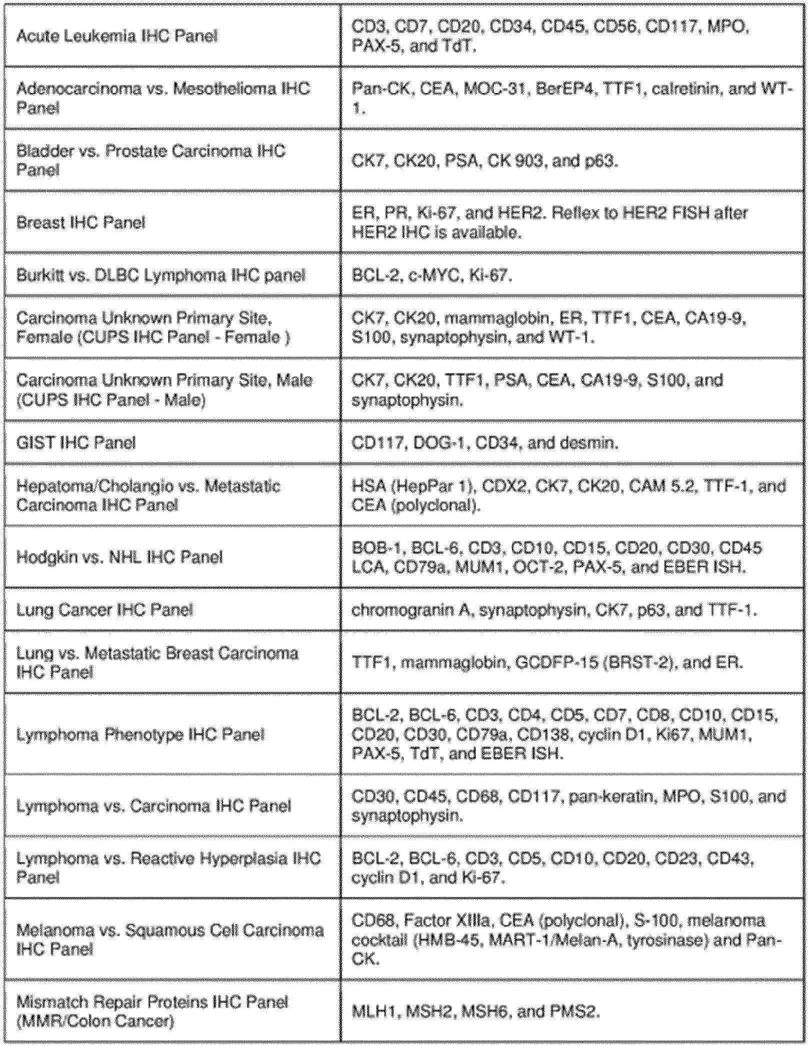

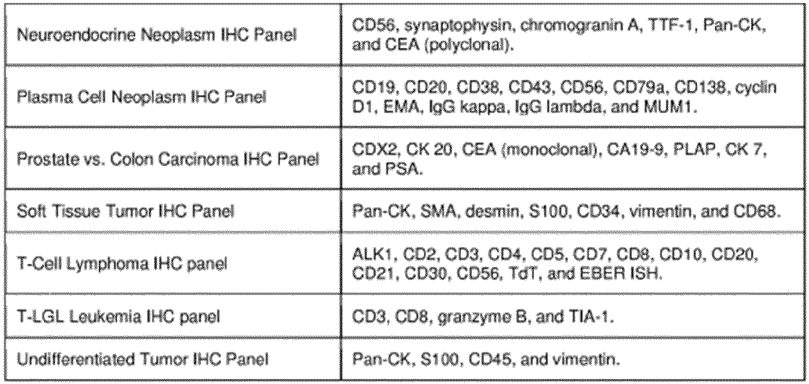

In some embodiments, the method may involve obtaining data (an image) as described above (an electronic form of which may have been forwarded from a remote location), and the image may be analyzed by a doctor or other medical professional to determine whether a patient has abnormal cells (e.g., cancerous cells) or which type of abnormal cells are present. The image may be used as a diagnostic to determine whether the subject has a disease or condition, e.g., a cancer. In certain embodiments, the method may be used to determine the stage of a cancer, to identify metastasized cells, or to monitor a patient's response to a treatment, for example.

The compositions and methods described herein can be used to diagnose a patient with a disease. In some cases, the presence or absence of a biomarker in the patient's sample can indicate that the patient has a particular disease (e.g., a cancer). In some cases, a patient can be diagnosed with a disease by comparing a sample from the patient with a sample from a healthy control. In this example, a level of a biomarker, relative to the control, can be measured. A difference in the level of a biomarker in the patient's sample relative to the control can be indicative of disease. In some cases, one or more biomarkers are analyzed in order to diagnose a patient with a disease. The compositions and methods of the disclosure are particularly suited for identifying the presence or absence of, or determining expression levels, of a plurality of biomarkers in a sample.

In some cases, the compositions and methods herein can be used to determine a treatment plan for a patient. The presence or absence of a biomarker may indicate that a patient is responsive to or refractory to a particular therapy.

In some cases, the method may be employed in a variety of diagnostic, drug discovery, and research applications that include, but are not limited to, diagnosis or monitoring of a disease or condition (where the image identifies a marker for the disease or condition), discovery of drug targets (where the a marker in the image may be targeted for drug therapy), drug screening (where the effects of a drug are monitored by a marker shown in the image), determining drug susceptibility (where drug susceptibility is associated with a marker) and basic research (where is it desirable to measure the differences between cells in a sample).

In certain embodiments, two different samples may be compared using the above methods. The different samples may be composed of an "experimental" sample, i.e., a sample of interest, and a "control" sample to which the experimental sample may be compared. In many embodiments, the different samples are pairs of cell types or fractions thereof, one cell type being a cell type of interest, e.g., an abnormal cell, and the other a control, e.g., normal, cell. If two fractions of cells are compared, the fractions are usually the same fraction from each of the two cells. In certain embodiments, however, two fractions of the same cell may be compared. Exemplary cell type pairs include, for example, cells isolated from a tissue biopsy (e.g., from a tissue having a disease such as colon, breast, prostate, lung, skin cancer, or infected with a pathogen, etc.) and normal cells from the same tissue, usually from the same patient; cells grown in tissue culture that are immortal (e.g., cells with a proliferative mutation or an immortalizing transgene), infected with a pathogen, or treated (e.g., with environmental or chemical agents such as peptides, hormones, altered temperature, growth condition, physical stress, cellular transformation, etc.), and a normal cell (e.g., a cell that is otherwise identical to the experimental cell except that it is not immortal, infected, or treated, etc.); a cell isolated from a mammal with a cancer, a disease, a geriatric mammal, or a mammal exposed to a condition, and a cell from a mammal of the same species, preferably from the same family, that is healthy or young; and differentiated cells and non-differentiated cells from the same mammal (e.g., one cell being the progenitor of the other in a mammal, for example). In one embodiment, cells of different types, e.g., neuronal and non-neuronal cells, or cells of different status (e.g., before and after a stimulus on the cells) may be employed. In another embodiment of the invention, the experimental material contains cells that are susceptible to infection by a pathogen such as a virus, e.g., human immunodeficiency virus (HIV), etc., and the control material contains cells that are resistant to infection by the pathogen. In another embodiment, the sample pair is represented by undifferentiated cells, e.g., stem cells, and differentiated cells.

The images produced by the method may be viewed side-by-side or, in some embodiments, the images may be superimposed or combined. In some cases, the images may be in color, where the colors used in the images may correspond to the labels used.

Cells from any organism, e.g., from bacteria, yeast, plants and animals, such as fish, birds, reptiles, amphibians and mammals may be used in the subject methods. In certain embodiments, mammalian cells, i.e., cells from mice, rabbits, primates, or humans, or cultured derivatives thereof, may be used.

### EXAMPLES

To further illustrate some embodiments of the present invention, the following specific examples are given with the understanding that they are being offered to illustrate examples of the present invention and should not be construed in any way as limiting its scope.

### MATERIALS AND METHODS

### Antibody and tissue preparation

The following oligonucleotides and antibodies were used:
*Keratin 5*/*14 assay*
   Left target oligonucleotide (conjugate arm): /5AzideN/TTTUUUGTAGUCACAUGACGTUGCCGACCGTCUGTT (SEQ ID NO: 1) conjugated to Rabbit monoclonal [EP1601Y] to Cytokeratin 5 (ab214586, Abcam)
   Right target oligonucleotide (conjugate arm): /5Phos/TCGACUACAC*C*A*A*A*TCGTGCGAAGTACGGATTTTTT/3AzideN/ (SEQ ID NO:2) conjugated to Rabbit monoclonal [SP53] to Cytokeratin 14 (ab236439, Abcam)
   Splint:/SPhos/GTGTAGTCGAAACAGACGGT (SEQ ID NO:3)
      Left reporter oligonucleotide: 
/5Phos/CGGCAACGTCATGTGACTACAACCCATTGTTCCGCAAGCGCGGGCCGCAAAT
AGCTCTTT/3Bio/ (SEQ ID NO:4)
      Right reporter oligonucleotide: 
A*A*T*GTTTCGCGTGCATCCGGCTCCACCGGATTTGCAGCTTTCCGTACTTCGCACG
ATTTG (SEQ ID NO:5)
   *CD20 assay*
      Left target oligonucleotide (conjugate arm): /5AzideN/TTTUUUCGAAAUAUGGAUCCCACGCCTGAACAUACG (SEQ ID NO:6) conjugated to Anti-human CD20 L26 (14-0202-82, ThermoFisherScientific)
      Right target oligonucleotide (conjugate arm): /5Phos/CGTGGUAGTCC*C*G*T*G*GTTCGGACCTAAATCTTTTTT/3 AzideN/ (SEQ ID NO:7) conjugated to Rabbit monoclonal [SP32] to CD20 (ab236434, Abcam)
      Splint:
         /5Phos/GACTACCACGCGTATGTTCA (SEQ ID NO:8)
      Left reporter oligonucleotide: 
/5Phos/GGCGTGGATCCATATTTCGAAATGCCCGATCGCAAGCGTTTGGCATGCGAC
GCCAACTT/3Bio/ (SEQ ID NO:9)
      Right reporter oligonucleotide: 
T*T*T*GAAGCAATCCGTGGCGGCGCAAACGTTTGTCGACAGATTTAGGTCCGAA
CCACGG (SEQ ID NO:10)
   *PCNA assay*
      Left target oligonucleotide (conjugate arm): /5AzideN/TTTUUUTATUAGCACGCCGGAAAGACAGAAAUGGAT (SEQ ID NO:11) conjugated to Rabbit monoclonal [EPR3821] to PCNA (ab218310, Abcam)
      Right target oligonucleotide (conjugate arm): /5Phos/CCUCGTAGG*G*A*A*T*GGGCGTTCACTCTTGTATTTTTT/3AzideN/ (SEQ ID NO:12) conjugated to Purified anti-human/mouse/rat PCNA (307902, Biolegend)
      Splint: /5Phos/CCCTACGAGGATCCATTTCT (SEQ ID NO:13)
      Left reporter oligonucleotide: 
/5Phos/GTCTTTCCGGCGTGCTAATAACACGTTGCGCAAACCATCGCAAACCAGTTGG
CGCATCGT/3Bio/ (SEQ ID NO:14)
      Right reporter oligonucleotide: 
A*A*T*GTTTCGCGTGCATCCGGCTCCACCGGATTTGCAGCTTTACAAGAGTGAACGC
CCATT (SEQ ID NO:15)
      Oligonucleotide modifications are listed below:
         /5AzideN/: Azide modification attached via NHS ester
         /5Phos/: Phosphorylation
         U: Deoxyuridine
         *: Phosphorothioate bond
         /3AzideN/: Azide modification attached via NHS ester
         /3Bio/: Biotin

**Antibody-oligonucleotide conjugation:** Antibodies containing sodium azide (NaN₃) were buffer exchanged to DPBS using 0.5 mL Zeba^{™} Spin Desalting Columns 7K MWCO. Amicon^{®} Ultra-0.5 Centrifugal Filter 10K MWCO devices were used to concentrate antibodies to 1 mg/ml, if needed.

DBCO - NHS-ester (cat no 761524, Sigma-Aldrich) was dissolved and diluted to 2 mM in DMSO. A 15-fold molar excess of DBCO-NHS ester was added to the antibody, and the reaction was incubated for 45 minutes at RT protected from light. 1M Tris-HCl pH 8 was added to a final concentration of 30-100 mM, and the reaction was incubated for 5 min at RT. A 0.5 mL Zeba^{™} Spin Desalting Column 7K MWCO, (Thermo Scientific cat. no 89882) equilibrated to DPBS was used according to manufacturer's instructions to remove unreacted DBCO-NHS ester. A 2.5-fold molar excess of azide-modified DNA oligonucleotide was added to the antibody activated with DBCO. The reaction was incubated in a fridge (2-8°C) for at least 60 hours. Successful conjugation was verified with polyacrylamide gel electrophoresis, staining conjugates with SYBR Gold Nucleic Acid Gel stain (S11494, Invitrogen) and InstantBlue Coomassie Protein Stain (Abcam, ab119211). Antibody-oligonucleotide conjugates were diluted to 0.15 µg/µl in DPBS with 0.1% BSA and 0.02% NaN₃.

**Tissue preparation:** FFPE blocks or tissue microarrays with human tonsil were sectioned in 4µm thick sections, and placed on TOMO glass slides (Matsunami). After baking, the slides were deparaffinized in xylene (2 times for 5 min) and hydrated in a series of graded ethanol to deionized water. Endogenous peroxidases were blocked with 3% H₂O₂ in PBS for 10 min at RT. The slides were rinsed 1 time in PBS. For antigen retrieval Antigen Retrieval Buffer, Citrate Buffer, pH 6.0 [Abcam, ab93678] was used for 50 min at 98°C. The slides were rinsed 1 time in PBS. A barrier was created by drawing with an ImmEdge^{™} hydrophobic barrier pen. Finally, the slides were rinsed in TBS with 0.05% Tween-20.

**Singleplex proximity ligation assay (PLA) for chromogenic detection in tissue**

**Tissue blocking:** The avidin blocking buffer was prepared as follows: TBS, 0.05% Tween-20, 10 mg/ml BSA, 0.5 mg/ml salmon sperm DNA (Sigma), avidin 5µg/ml.

Avidin blocking buffer was applied to cover the tissue section and the slides were incubated for 1 h at RT. Finally, 2 washes of 2 min in TBS with 0.05% Tween-20 were performed.

The biotin blocking buffer was prepared as follows: TBS, 0.05% Tween-20, 10 mg/ml BSA, 0.5 mg/ml salmon sperm DNA (Sigma), 2mM D-biotin.

Biotin blocking buffer was applied to cover the tissue section and the slides were incubated for 30 min at RT. The slides were rinsed once with in TBS with 0.05% Tween-20.

**Antibody incubation:** A pair of antibody-oligonucleotide conjugates were diluted to 1 µg/ml of each conjugated antibody in biotin blocking buffer. The diluted conjugates were applied to the slides. The slides were incubated at 4°C overnight. Slides were washed 2 times for 5 min in TBS with 0.05% Tween-20.

**Proximity ligation assay (PLA):** The two target oligonucleotides were ligated by adding 125nM splint, 0.04 U/µl T4 DNA ligase (ThermoScientific), 10mM tris acetate, 10mM magnesium acetate, 50mM potassium acetate, 0.5mg/ml BSA, 200mM NaCl, and 0.05% Tween-20. The reaction was incubated for 30 min at 37°C in a humidity chamber. This splint templated ligation step was omitted for the no ligation negative control. The slides were washed 2 times for 5 min in TBS with 0.05% Tween-20.

Reporter oligonucleotides, one with a biotin (left) and one without (right), were diluted to 33nM in 10mM tris acetate, 10mM magnesium acetate, 50mM potassium acetate, 0.5mg/ml BSA, 250mM NaCl, and 0.05% Tween-20, and then added to the slides to hybridize to the first ligation products. The hybridization reaction was incubated for 30 min at 37°C in a humidity chamber. The slides were then washed 2 times for 5 min in TBS with 0.05% Tween-20. The reporter oligonucleotides were then ligated by adding 0.04 U/µl T4 DNA ligase (ThermoScientific), 10mM tris acetate, 10mM magnesium acetate, 50mM potassium acetate, 0.5mg/ml BSA, 200mM NaCl, and 0.05% Tween-20, during a 30 min incubation at 37°C in a humidity chamber. The slides were washed 2 times for 2 min in TBS with 0.05% Tween-20.

Unligated reporter oligonucleotides were digested, and ligated reaction products/reporter probes were prepared for release with a nuclease mix containing 0.01 U/µl USER (New England Biolabs), 0.1 U/µl Lambda exonuclease (New England Biolabs), 1X rCutSmart buffer (New England Biolabs) and 0.05% Tween-20. The slides were washed 2 times for 5 min in TBS with 0.05% Tween-20.

**Chromogenic detection of PLA in tissue:** Chromogenic detection was used to visualize the biotinylated ligated reporter molecules in tissue sections after PLA (Figure 1, 2). The slides were incubated with 3.2 µg/ml streptavidin conjugated horse radish peroxidase (S2438, Sigma Aldrich) in TBS with 0.25 mg/ml BSA for 20 min in a humidity chamber at RT. The slides were washed 2 times for 5 min in TBS. Betazoid DAB chromogen kit (Biocare Medical) was used according to the manufacturer's instructions. Nuclear counterstaining was performed with Mayer's hematoxylin (HTX) (Histolab). The slides were dehydrated in graded series of ethanol and xylene, and then mounted to cover slips with Pertex (Histolab, cat no: 00811).

**Imaging:** The slides were imaged with brightfield settings on Pannoramic ScanII slide scanner (3D Histech) according to the manufacturer's instructions. ImageJ was used for color deconvolution to extract only the DAB staining of the ligated reporter molecules and remove the HTXstaining.

### Multiplex proximity ligation assay (PLA) with hybridization chain reaction based fluorescent detection in tissue

### Tissue blocking: As described above

**Antibody incubation:** Multiple pairs of antibody-oligonucleotide conjugates were diluted to 1 µg/ml of each conjugated antibody in biotin blocking buffer. The diluted conjugates were applied to the slides. The slides were incubated at 4°C overnight. Slides were washed 2 times for 5 min in TBS with 0.05% Tween-20.

**Proximity ligation assay (PLA):** Pairs of target oligonucleotides were ligated by adding 125nM of each assay specific splint, 0.04 U/µl T4 DNA ligase (ThermoScientific), 10mM tris acetate, 10mM magnesium acetate, 50mM potassium acetate, 0.5mg/ml BSA, 200mM NaCl, and 0.05% Tween-20. The reaction was incubated for 30 min at 37°C in a humidity chamber. This splint templated ligation step was omitted for the no ligation negative control (fig 3). The slides were washed 2 times for 5 min in TBS with 0.05% Tween-20.

Reporter oligonucleotides, 2 unique sequences for each PLA pair, were diluted to 33nM in 10mM tris acetate, 10mM magnesium acetate, 50mM potassium acetate, 0.5mg/ml BSA, 250mM NaCl, and 0.05% Tween-20, and then added to the slides to hybridize to the first ligation products. The hybridization reaction was incubated for 30 min at 37°C in a humidity chamber. The slides were then washed 2 times for 5 min in TBS with 0.05% Tween-20. The reporter oligonucleotides were then ligated by adding 0.04 U/µl T4 DNA ligase (ThermoScientific), 10mM tris acetate, 10mM magnesium acetate, 50mM potassium acetate, 0.5mg/ml BSA, 200mM NaCl, and 0.05% Tween-20, during a 30 min incubation at 37°C in a humidity chamber. The slides were washed 2 times for 2 min in TBS with 0.05% Tween-20.

Unligated reporter oligonucleotides were digested, and ligated reaction products/reporter probes were prepared for release with a nuclease mix containing 0.01 U/µl USER (New England Biolabs), 0.1 U/µl Lambda exonuclease (New England Biolabs), 1X rCutSmart buffer (New England Biolabs) and 0.05% Tween-20. The slides were washed 2 times for 5 min in TBS with 0.05% Tween-20.

**HCR detection of reporter molecules on cover slips:** The slides were washed 2 min with 2x SSC with 0.1% Tween-20. Probes with HCR initiator sequences, recognizing the reaction products/reporter probes were diluted to 10 nM in 4X SSC with 20% ethylene carbonate and 0.1 % Tween-20, and added to the tissue slides. The slides were incubated for 1 hour in a humidity chamber at RT, and then washed 2 times for 5 min in 2X SSC with 0.1% Tween-20. HCR was performed as previously described by Choi (ACS Nano 2014 8: 4284-94). Briefly one pair of HCR hairpin probes with ATTO565 and one pair of probes with ATTO647N were individually diluted to 0.5 µM in 40 µl 5X SSC, incubated at 95°C for 5 min, and then allowed to cool down at RT for 10 min. Thereafter the four hairpin probes were mixed and diluted to 10 nM in 5X SSC with 0.1% Tween-20. The HCR hairpin probe mix was applied to the tissue slides, and the reaction was allowed to proceed for 3 h at RT protected from light in a humidity chamber. The slides were washed once with 2X SSC with 0.1% Tween-20 and once with TBS. Nuclei were stained with 20 µM Hoechst in 1x TBS for 10 min at RT protected from light in a humidity chamber. The slides were washed 2x2 min in 1X TBs before they were mounted with SlowFade Diamond Antifade Mountant (Invitrogen) and cover slips.

**Imaging:** The slides were imaged with Pannoramic ScanII slide scanner (3D Histech) according to the manufacturer's instructions, using DAPI, TRITC and Cy5 filters.

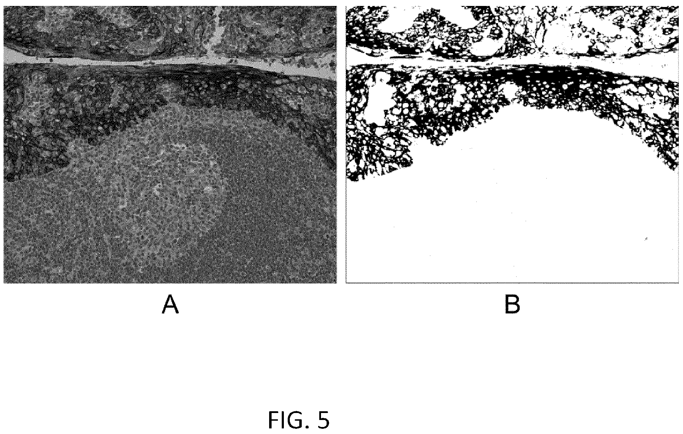

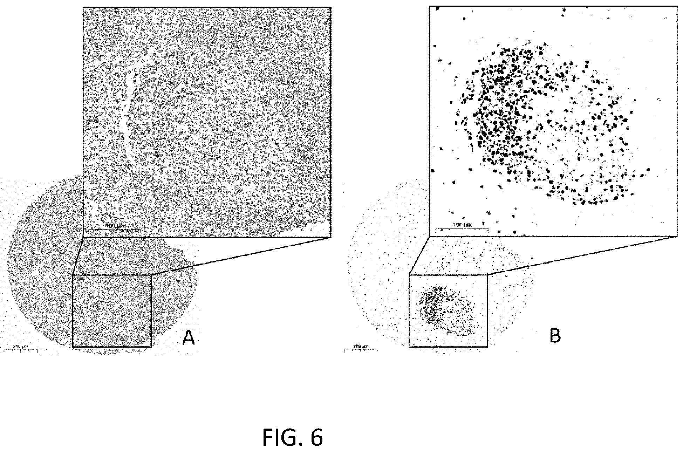

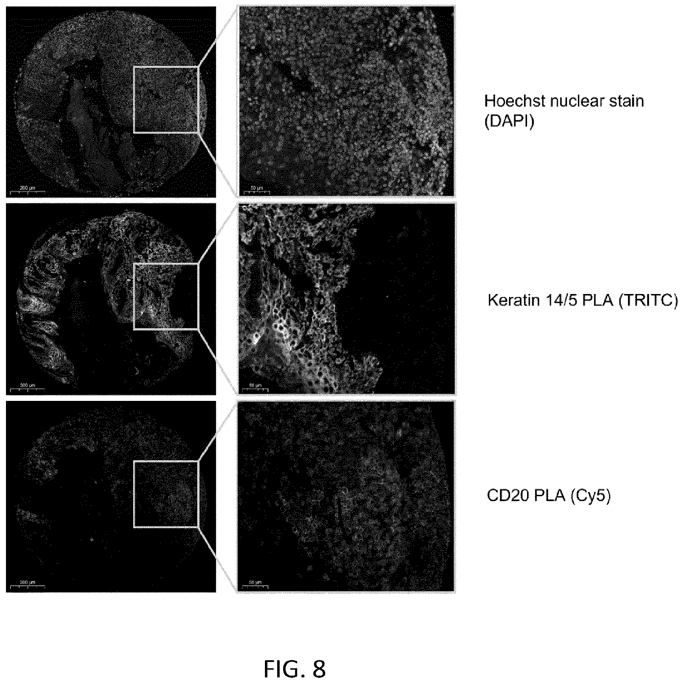

Exemplary results are shown in Figs. 5-8. Fig. 5 and 6 demonstrate that one can detect cytoplasmic (Fig. 5) and nuclear (Fig. 6) proteins with a chromogenic singleplex PLA detection (Fig. 4) in human FFPE samples. Fig. 7 illustrates that one only detect a PLA signal when T4 DNA ligase has been added to the reaction. Fig. 8 illustrates that one can run duplex protein detection by PLA if one uses a fluorescence readout to detect unique oligonucleotide sequences for different targets.

## Claims

1. A method for performing an *in situ* proximity assay, comprising:
(a) performing a proximity assay on one or more pairs of binding agent-oligonucleotide conjugates that are bound to the sample, *in situ,* to produce proximity assay reaction products; and
(b) labeling the sites in the sample at which the proximity assay reaction products are produced, wherein the labeling comprises hybridizing an oligonucleotide to the proximity assay reaction products and does not involve primer extension;
wherein the method further comprises treating the sample with one or more exonucleases between steps (a) and (b), thereby removing any nucleic acids that have free 3' and/or 5' ends.

2. A method as claimed in claim 1, wherein the one or more exonucleases comprise at least a 5' exonuclease and a 3' exonuclease.

3. A method as claimed in claim 1 or 2, wherein the proximity assay is a proximity ligation assay.

4. A method as claimed in claim 1, 2 or 3, wherein in (b) the oligonucleotide hybridizes across a ligation junction.

5. A method as claimed in claim 1 or 2, wherein the proximity assay of (a) is a proximity extension assay.

6. A method as claimed in claim 5, wherein some capture agents used in the assay are linked to primers and some of the capture agents are linked to templates that include one or more uracils.

7. A method as claimed in claim 6, wherein the method further comprises cleaving the templates at the uracils, between steps (a) and (b).

8. A method as claimed in any preceding claim, wherein the oligonucleotide of (b) is labeled.

9. A method as claimed in claim 8, wherein the oligonucleotide of (b) is biotinylated.

10. A method as claimed in claim 9, wherein step (b) comprises incubating the sample with a streptavidin/avidin-peroxidase conjugate and a detectable substrate that is cleaved by the conjugate.

11. A method as claimed in claim 10, wherein the labeling is done by an Avidin-Biotin Complex (ABC) method or a Labeled Streptavidin-Biotin (LSAB) method.

12. A method as claimed in claim 8, wherein the oligonucleotide is fluorescently labeled.

13. A method as claimed in any of claims 1 to 7, wherein:
the oligonucleotide is unlabeled and has a tail that does not hybridize to the proximity assay reaction products, and
the method comprises detecting the tail.

14. A method as claimed in claim 13, wherein the tail of the oligonucleotide is detected by hybridization chain reaction.

15. A method as claimed in any preceding claim, wherein the method further comprises:
(c) imaging the sample.

16. A method as claimed in any preceding claim, wherein the biological sample is a tissue section.

## Patentansprüche

1. Verfahren zur Durchführung eines *in situ* Proximity-Assays, umfassend:
(a) Durchführen eines Proximity-Assays an einem oder mehreren Paaren von Bindemittel-Oligonukleotid-Konjugaten, die an die Probe *in situ* gebunden sind, um Proximity-Assay-Reaktionsprodukte zu erzeugen; und
(b) Labeln der Stellen in der Probe, an denen die Proximity-Assay-Reaktionsprodukte erzeugt werden, wobei das Markieren das Hybridisieren eines Oligonukleotids an die Proximity-Assay-Reaktionsprodukte umfasst und keine Primer Extension beinhaltet;
wobei das Verfahren ferner das Behandeln der Probe mit einer oder mehreren Exonukleasen zwischen den Schritten (a) und (b) umfasst, wodurch alle Nukleinsäuren mit freien 3' und/oder 5'-Enden entfernt werden.

2. Verfahren nach Anspruch 1, wobei die eine oder mehreren Exonukleasen mindestens eine 5'-Exonuklease und eine 3'-Exonuklease umfassen.

3. Verfahren nach Anspruch 1 oder 2, wobei der Proximity-Assay ein die Proximity-Ligation-Assay ist.

4. Verfahren nach Anspruch 1, 2 oder 3, wobei in (b) das Oligonukleotid über eine Ligationsverbindung hybridisiert.

5. Verfahren nach Anspruch 1 oder 2, wobei der Proximity-Assay von (a) ein Proximity-Extensions-Assay ist.

6. Verfahren nach Anspruch 5, wobei einige der im Assay verwendeten Capture-Mittel an Primer gebunden sind und einige der Capture-Mittel an Templates gebunden sind, die ein oder mehrere Uracile enthalten.

7. Verfahren nach Anspruch 6, wobei das Verfahren weiter ein Auftrennen der Templates an den Uracilen zwischen den Schritten (a) und (b) umfasst.

8. Verfahren nach einem der vorgehenden Ansprüche, wobei das Oligonukleotid aus Schritt (b) gelabelt wird.

9. Verfahren nach Anspruch 8, wobei das Oligonukleotid biotinyliert wird.

10. Verfahren nach Anspruch 9, wobei Schritt (b) weiter das inkubieren der Probe mittels Streptavidin/Avidin-Peroxidase-Konjugat und einem nachweisbaren Substrat, das durch das Konjugat gespalten wird.

11. Verfahren nach Anspruch 10, wobei das Labeln mittels eines Avidin-Biotin-Komplex (ABC)- Verfahrens oder eines Labeled Streptavidin-Biotin (LSAB)-Verfahrens erfolgt.

12. Verfahren nach Anspruch 8, wobei das Oligonukleotid fluoreszierend gelabelt wird.

13. Verfahren nach einem der Ansprüche 1 bis 7, wobei:
das Oligonukleotid ungelabelt ist und einen Schwanz hat, der nicht mit Proximity-Assay-Reaktionsprodukten hybridisiert, und
das Verfahren das Detektieren des Schwanzes umfasst.

14. Verfahren nach Anspruch 13, wobei der Schwanz des Oligonukleotids durch eine Hybridisierungskettenreaktion detektiert wird.

15. Verfahren nach einem der vorgehenden Ansprüche, wobei das Verfahren weiter umfasst:
(c) Imaging der Probe.

16. Verfahren nach einem der vorgehenden Ansprüche, wobei die biologische Probe eine Gewebeprobe ist.

## Revendications

1. - Procédé pour effectuer un essai de proximité *in situ,* comprenant :
(a) effectuer un essai de proximité sur une ou plusieurs paires de conjugués agent de liaison - oligonucléotide qui sont liés à l'échantillon, *in situ,* pour donner des produits de réaction d'essai de proximité ; et
(b) marquer les sites dans l'échantillon au niveau desquels les produits de réaction d'essai de proximité sont obtenus, le marquage comprenant l'hybridation d'un oligonucléotide aux produits de réaction d'essai de proximité et n'impliquant pas d'extension d'amorce ;
le procédé comprenant en outre le traitement de l'échantillon par une ou plusieurs exonucléases entre les étapes (a) et (b), permettant ainsi d'éliminer tout acide nucléique qui a des extrémités 3' et/ou 5' libres.

2. - Procédé selon la revendication 1, dans lequel la ou les exonucléases comprennent au moins une 5' exonucléase et une 3' exonucléase.

3. - Procédé selon l'une des revendications 1 ou 2, dans lequel l'essai de proximité est un essai de ligature de proximité.

4. - Procédé selon l'une des revendications 1, 2 ou 3, dans lequel, en (b), l'oligonucléotide s'hybride à travers une jonction de ligature.

5. - Procédé selon l'une des revendications 1 ou 2, dans lequel l'essai de proximité de (a) est un essai d'extension de proximité.

6. - Procédé selon la revendication 5, dans lequel certains agents de capture utilisés dans l'essai sont liés à des amorces et certains des agents de capture sont liés à des matrices qui comprennent un ou plusieurs uraciles.

7. - Procédé selon la revendication 6, dans lequel le procédé comprend en outre le clivage des matrices au niveau des uraciles, entre les étapes (a) et (b).

8. - Procédé selon l'une quelconque des revendications précédentes, dans lequel l'oligonucléotide de (b) est marqué.

9. - Procédé selon la revendication 8, dans lequel l'oligonucléotide de (b) est biotinylé.

10. - Procédé selon la revendication 9, dans lequel l'étape (b) comprend l'incubation de l'échantillon avec un conjugué streptavidine/avidine-peroxydase et un substrat détectable qui est clivé par le conjugué.

11. - Procédé selon la revendication 10, dans lequel le marquage est effectué par une méthode ABC (complexe avidine-biotine) ou une méthode LSAB (streptavidine-biotine marquée).

12. - Procédé selon la revendication 8, dans lequel l'oligonucléotide est marqué par fluorescence.

13. - Procédé selon l'une quelconque des revendications 1 à 7, dans lequel :
l'oligonucléotide est non marqué et a une queue qui ne s'hybride pas aux produits de réaction d'essai de proximité ; et
le procédé comprend la détection de la queue.

14. - Procédé selon la revendication 13, dans lequel la queue de l'oligonucléotide est détectée par réaction en chaîne d'hybridation.

15. - Procédé selon l'une quelconque des revendications précédentes, dans lequel le procédé comprend en outre :
(c) imager l'échantillon.

16. - Procédé selon l'une quelconque des revendications précédentes, dans lequel l'échantillon biologique est une coupe de tissu.
